# EUROPEAN PATENT APPLICATION

(11) **EP 4 663 140 A1**
(43) Date of publication of application: **17.12.2025**
(21) Application number: 24182099.2
(22) Date of filing: 13.06.2024
(51) Int. Cl.: A61B 17/86, A61B 17/70, A61B 17/88

(54) **SCREW ELEMENT FOR USE IN SPINAL, ORTHOPAEDIC OR TRAUMA SURGERY**

(71) Applicant: Biedermann Technologies GmbH & Co. KG, 78166 Donaueschingen (DE)
(72) Inventor: BIEDERMANN, Lutz, 78048 VS-Villingen (DE); BIEDERMANN, Timo, 78647 Trossingen (DE); FISCHER, Bernd, 79877 Friedenweiler (DE)
(74) Representative: Prüfer & Partner mbB Patentanwälte · Rechtsanwälte

(57) **Abstract**

A screw element is provided for use in spinal, orthopaedic or trauma surgery, the screw element (1) including a screw axis (S) and a screw thread (3) and a drive portion (6) comprising a recess (7) with a wall (9) that defines a main contour configured to engage with a screwdriver, the wall further defining a plurality of drive grooves (8), wherein the drive grooves (8) are spaced apart from each other in a circumferential direction around the screw axis (S); and wherein a wall portion (9a) of the recess (7) comprises a protrusion (10) that protrudes from the main contour into the recess (7).

## Description

The invention relates to a screw element for use in Spinal, Orthopaedic or trauma surgery. In particular, the screw element can be a screw element of a bone anchoring device configured to be connected to a rod, a screw element of a bone plate or a set screw to be used in such devices.

In spinal, orthopaedic or trauma surgery the insertion of screw elements into bone or the use of screw elements such as a set screw requires precision. Often either the screw element is a tiny element or the available space for handling it is reduced. Therefore, it is important to avoid loss of the screw element in a patient's body and/or to safely tighten the screw element.

US 8,867,639 B2 describes a screw element for use in spinal, orthopaedic or trauma surgery, the screw element including a screw axis and a screw thread, a drive portion for engagement with a screwdriver, wherein the drive portion comprises a first recess with a wall and substantially longitudinally extending drive grooves formed in the wall, a second recess between a free end of the screw element and the first recess, wherein the second recess has a wall and a gradually increasing inner diameter towards the free end, and wherein in the wall of the second recess guide grooves are formed at positions corresponding to the position of the drive grooves that allow an engagement portion of a screwdriver to be guided to engage the drive grooves. This screw element is, for example, useful in minimally invasive surgery as it facilitates the insertion of the screwdriver in the drive portion.

There is still a need for further improving the handling of a screw element with a screwdriver, in particular in view of the above-mentioned clinical applications.

It is therefore an object of the invention to provide a screw element and a system of a screw element and a screwdriver that facilitates the handling thereof. It is also an object to provide a method for manufacturing such a screw element.

The object is solved by a screw element according to claim 1, by a system of a screw element and a screwdriver according to claim 14 and by a method of manufacturing the screw element according to claim 15. Further developments of the screw element are given in the dependent claims.

According to an aspect of the invention a screw element for use in spinal, orthopaedic or trauma surgery is provided, the screw element including a screw axis and a screw thread and a drive portion comprising a recess with a wall that defines a main contour configured to engage with a screwdriver, the wall further defining a plurality of drive grooves, wherein the drive grooves are spaced apart from each other in a circumferential direction around the screw axis; and wherein a wall portion of the recess comprises a protrusion that protrudes from the main contour into the recess.

The main contour may be considered, for example, as the wall portions which correspond to the shape of the engagement portion of a screwdriver so that a form-fit connection can be established.

The screw element provides for an enhanced clamping of the engagement portion of a screwdriver. This allows a safe holding of the screw element when the screw element is screwed into bone or when the screw element is used as a set screw.

Moreover, the screw element allows to apply a force more efficiently to screw-in the screw element, as a radial play between the engagement portion of the screwdriver and the drive portion of the screw element may be reduced.

The drive portion can have various shapes, such as a polygonal shape or a star-shape (torx-shape) or any other shape suitable for providing a form fit engagement with an engagement portion of a screwdriver.

In another aspect, the screw element may be used as part of a polyaxial bone anchoring device. The polyaxial bone anchoring element comprises the screw element and a receiving part in which the head of the screw element is pivotably held and can be locked at an angle that the screw axis forms with a central axis of the receiving part. In the head of the screw element, the drive portion with the protrusion in a wall portion may be provided. The receiving part further comprises a recess for receiving a rod. The rod can be fixed relative to the receiving part by means of a screw element in the form of a set screw which comprises the drive portion with the protrusion in the wall portion.

In another aspect, the screw element may be used together with a bone plate. The screw element is configured to fix the bone plate to bone. In the head of the screw element, the drive portion with the protrusion in a wall portion may be provided. The head of the screw element may be held in a seat provided around a through-hole in the bone plate through which the shank of the screw element extends. The head may be secured against pull-out in the seat by a screw element in the form of a set screw which comprises the drive portion with the protrusion in the wall.

According to a further aspect, the screw element is manufactured with an additive manufacturing technique, such as laser sintering, laser melting, electron beam melting, fused deposition molding (FDM) or others. With such a technique any three-dimensional shape can be manufactured based on CAD-data of the screw element, especially any complicated shapes that cannot or can only hardly be manufactured with subtractive methods. The manufacturing can be carried out on demand for a specific patient's application.

At least a portion of the screw element that comprises the drive portion with the protrusion in a wall portion may have a size and a shape such that it can most efficiently only be manufactured by an additive manufacturing method.

The use of an additive manufacturing method allows to easily design various screw elements with different shapes of the protrusion. By means of this, various holding forces of the engagement portion of the screwdriver and the drive portion of the screw element can be achieved, for example.

Further features and advantages will become apparent from the description of embodiments by means of the accompanying drawings. In the drawings:
- Fig. 1:: shows a perspective view of a screw element according to a first embodiment together with a front portion of a screwdriver prior to attaching the screwdriver to the screw element.
- Fig. 2:: shows an enlarged view of a portion of Fig. 1.
- Fig. 3:: shows a cross-sectional view of a portion of the screw element of Figs. 1 and 2 including a head of the screw element and an engagement portion of the screwdriver of Figs. 1 and 2, the cross-section taken in a plane including the screw axis of the screw element and the longitudinal axis of rotation of the screwdriver.
- Fig. 4:: shows a cross-sectional view of the screw element and the engagement portion of Fig. 3 in the same plane with the engagement portion of the screwdriver engaging the screw element.
- Fig. 5:: shows an enlarged portion of Fig. 4.
- Fig. 6:: shows another perspective view of a portion of the screw element of Figs. 1 to 5 including the head of the screw element.
- Fig. 7:: shows a perspective view of a modified first embodiment of the screw element including the head of the screw element.
- Fig. 8:: shows a perspective view of another modified first embodiment of the screw element including the head of the screw element.
- Fig. 9:: shows a perspective view of a further modified first embodiment of the screw element including the head of the screw element.
- Fig. 10:: shows a perspective view of a polyaxial bone anchoring device with a second embodiment of the screw element in the form of a set screw.

- Fig. 11:: shows an enlarged perspective view of the second embodiment of the screw element of Fig. 10.
- Fig. 12:: shows a perspective view of a modified second embodiment of the screw element.
- Fig. 13:: shows a perspective view of a further modified second embodiment of the screw element.
- Fig. 14:: shows a perspective view of an application of the screw element according to the first embodiment as shown in Figs. 1 to 9.
- Fig. 15:: shows another application of the screw element according to the first embodiment shown in Figs. 1 to 9 and of the screw element according to the second embodiment shown in Figs. 10 to 13.
- Fig. 16:: shows a still further application of the screw element of Figs. 1 to 9.

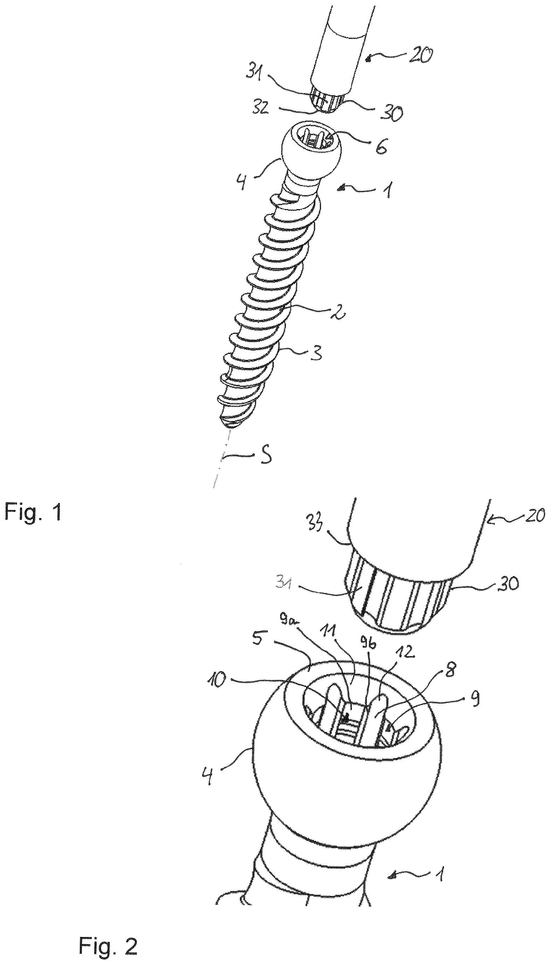

Referring to Figs. 1 to 6, a screw element 1 according to a first embodiment includes a shank 2 with a bone thread (or a screw thread) 3 on at least a portion of the shank 2 and a head 4. The shank 2 is configured to be inserted into a bone, for example, into a pedicle of a vertebra. A screw axis S is defined by the axis of the bone thread 3. The head 4 has a spherical segment shape and a free end 5 on a side opposite to the shank 2. A drive portion 6 that is configured to engage with an engagement portion 30 of a screwdriver 20 is provided at the free end 5. The drive portion 6 is explained in more detail below.

The drive portion 6 of the screw element 1 comprises a first recess 7 that is located at a distance from the free end 5 and that has an inner wall 9 defining a main contour suitable for engagement with the screwdriver. The wall is substantially cylindrical with a main inner diameter and with a cylinder axis coaxial with the screw axis S. A plurality of longitudinal drive grooves 8 are formed by the inner wall of the substantially cylindrical first recess 7. A cross-section of each of the drive grooves 8, taken along a plane perpendicular to the screw axis S, may be substantially a circular segment-shaped or otherwise rounded. From a top view, the drive grooves 8 are arranged circumferentially around the first recess 7 in a star-like manner. For example, an even number, such as six drive grooves 8 may be provided. In addition, the first recess 7 has a substantially flat bottom 7a. An axial depth of the first recess 7 is such that the engagement portion 30 of the screwdriver can be immersed therein. In particular, the depth of the first recess may substantially corresponds to an axial length of the engagement portion 30 of the screwdriver 20. Thus, the size of the first recess 7 with the drive groove 8 is sufficient for applying a necessary torque for inserting or advancing the screw element 1.

Between consecutive drive grooves 8 in a circumferential direction, there are wall portions 9a. In the embodiment shown, the wall portions 9a have a cylindrical shape due to the cylindrical main contour of the first recess 7. The drive grooves 8 may merge into the cylindrical wall portions 9a via slanted portions 9a.

On each wall portion 9a, a protrusion 10 is formed, that protrudes into the recess 7. In other words, the protrusion 10 protrudes from the main contour defined by the wall. The protrusion 10 comprises two axially spaced apart circumferential ribs 10a, 10b that extend along the entire wall portion 9 in the circumferential direction. An axial position of the ribs 10a, 10b may be substantially in the center of the wall portion 9a between the slanted portions 9b. A height of the protrusion 10 above the main contour is such that it permits to insert the engagement portion 30 of the screwdriver 20 but reduces a radial play of the engagement portion 30 within the first recess 7. In addition, the protrusion 10 has such a size and/or shape that it is suitable to hold the engagement portion 30 in the first recess 7 by friction. In a practical example, the height to which the protrusion protrudes above the main contour of the wall portion 9a is only a few hundredths of a millimeter. In the embodiment shown, each of the wall portions 9a has a protrusion 10, however, it may be sufficient that at least one of the wall portions 9a, preferably more than one wall portion, and still more preferably wall portions 9 that are arranged opposite to each other have the protrusion 10. The shape of the ribs 10a, 10b is rounded, in particular free of a sharp edge, such that the insertion of the engagement portion 30 can be effected smoothly, in particular without jamming.

Between the first recess 7 and the free end 5 there may be a second recess 11 that conically tapers and narrows from the free end 5 towards the first recess 7. A depth of the second recess 11 in the axial direction may be approximately one fifth to one third of the depth of the first recess 7. The second recess 11 provides an enlarged bevelled surface that facilitates insertion of the engagement portion 30 of the screwdriver 20 into the drive portion 6. A plurality of guide grooves 12 are provided in the wall defining the second recess 11 at positions corresponding to the positions of the drive grooves in the first recess 7. The guide grooves 12 have a size and a shape so that they merge into the drive grooves 8. Due to the bevelled surface of the second recess 11, the depth of the guide grooves 12 gradually increases from the free end 5 towards the drive groove 8. This allows for a more precise guiding of the engagement portion 30 of the screwdriver 20 into the first recess 7 while simultaneously facilitating the engagement between the engagement portion 30 and the drive portion 6.

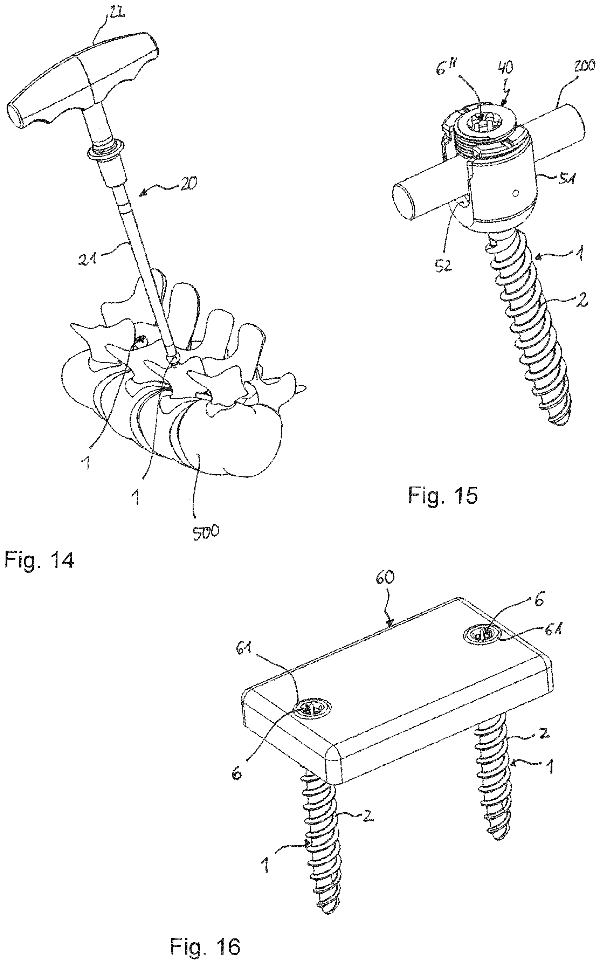

The screwdriver includes a drive shaft 21 and a handle 22 (Fig. 14) at one end of the drive shaft 21 and the engagement portion 30 at the opposite end of the drive shaft 21. The engagement portion 30 has a substantially cylindrical main contour that fits into the recess into the first recess 7. In addition, it has longitudinally extending rib-like engagement protrusions 31 that are sized to engage the drive grooves 8 to apply torque onto the screw element 1. The engagement portion may be bevelled towards a free end surface 32 of the engagement portion 30. Moreover, between the engagement portion 30 and the shaft portion, there may be a shoulder 33 for abutment at the free end 5 of the head 4.

The screw element may be made of any bio-compatible material, preferably, however, of titanium or stainless steel or of any other bio-compatible metal or metal alloy or plastic material. As a bio-compatible alloy, a NiTi-alloy, for example Nitinol, may be used. Other materials can also be magnesium or magnesium alloys. Bio-compatible plastic materials for use may be, for example, polyether ether ketone (PEEK) or poly-L-lactide acid (PLLA).

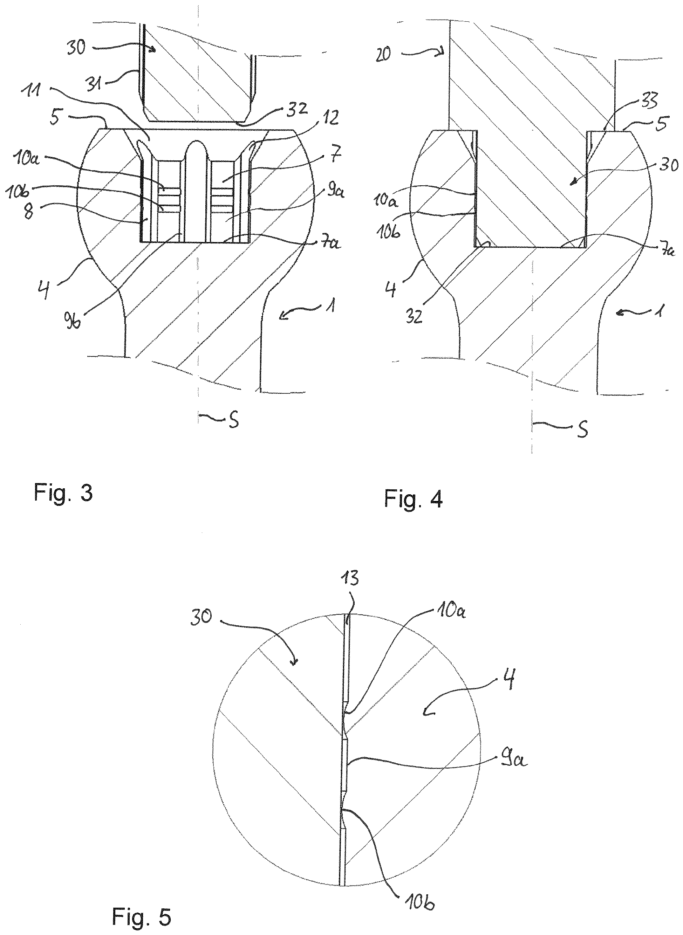

In use, as depicted in Figs. 3 to 5, when the engagement portion 30 is inserted into the drive portion 6, it enters the first recess 7 until its free end surface 32 abuts against the bottom 7a of the first recess 7. It should be noted that in Fig. 3 the engagement portion is oriented with its drive axis coaxial to the screw axis. If the drive axis is not coaxial to the screw axis prior to entering the recess 7, the guide grooves 12 guide the engagement portion 30 so that it aligns with the first recess 7 during insertion. As can be seen in Figs. 4 and 5, there is a radial gap 13 between the outer wall portions of the engagement portion 30 and the wall portions 9a except at the positions of the protrusion 10, i.e., at the positions of the ribs 10a, 10b. Hence, the protrusion 10 reduces the radial play and clamps the engagement portion so that it is firmly held in the first recess 7 by friction. By means of this, the screw element 1 can be held safely before it is inserted into bone. During screwing-in the screw element 1, the force applied can be transferred more efficiently.

In particular, the screw element as a whole or at least the portion with the drive portion 6 is manufactured using an additive manufacturing method, more specifically, an additive layer-manufacturing method. In such a method, the screw element is built up by layer-wise deposition of a building material and solidifying or melting the building material in each layer at positions corresponding to the cross-section of the screw element in the respective layer. A suitable method is, for example, selective laser-sintering (SLS) or selective laser-melting (SLM) in which the building material is a powder, such as a metal powder or a plastic powder, and a laser is used to melt the powder. In this method consecutive layers of the building material are applied to a support surface or a previous layer and the laser beam is steered to the positions of a layer which correspond to the cross-section of the screw element in each layer. Thus, the screw element is built up in an additive manner. The building material may preferably be a titanium powder or a stainless-steel powder. Alternatively, an electron beam may be used to melt the building material. Also, other known methods of powder-based three-dimensional printing in which layers of a powder material are deposited and solidified by applying a binder material at positions corresponding to the screw element may be used. Still further additive manufacturing methods, for example, fused deposition modelling (FDM) may also be used.

With this method it is possible to produce the screw element having the protrusions 10 in the drive portion 6. It should be mentioned that the screw element 1 can also be produced as a two-part device, wherein, for example, the shank is produced conventionally on a lathe and using a thread-cutting device whereas the head with the drive portion 6 is produced via an additive manufacturing method as described above. The two parts can then be assembled, for example by means of a press-fit connection or a threaded connection.

It shall be noted that the additive manufacturing method, in particular an additive layer-manufacturing method, influences the outer appearance of the screw element manufactured by such a method. For example, the layers may be visible on the surface of the finished object even if it is post-treated, such as polished, etched, coated or otherwise treated. It may also be possible to identify the traces of the laser or electron beam when inspecting the fabricated object. Hence, the use of an additive manufacturing method, in particular the additive layer-manufacturing method, can be distinguished based on the finished object compared to a conventional subtractive manufacturing method.

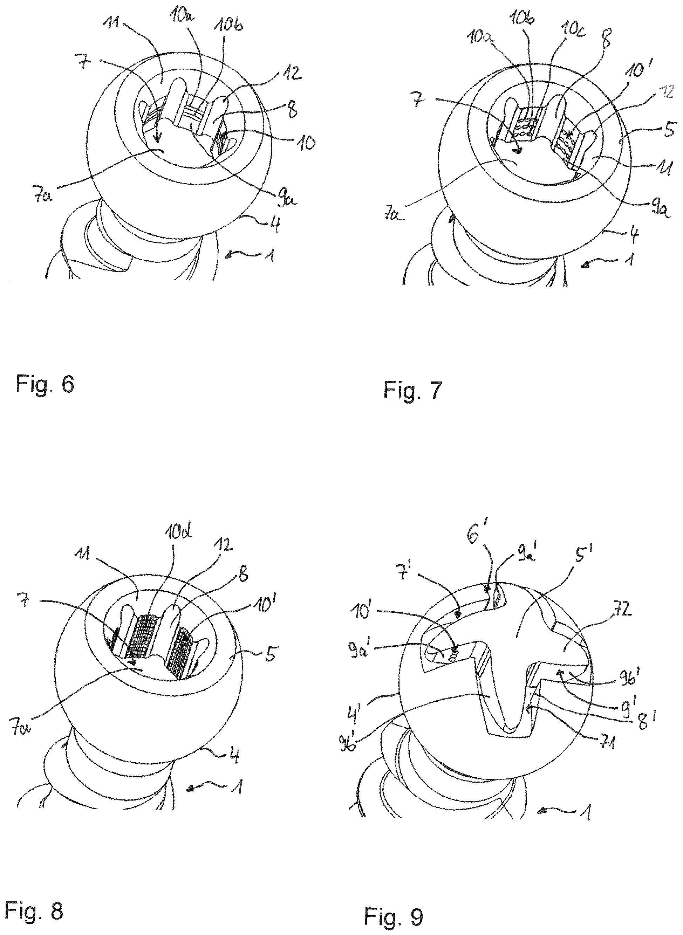

Fig. 6 depicts the screw element according to the first embodiment from another perspective view. In particular, the bottom 7a of the first recess is shown. Referring further to Figs. 7 to 9, various modifications of the first embodiment of the screw element are shown. Parts and portions that are identical or highly similar are marked with the same reference numerals as in the first embodiment and the description thereof will not be repeated.

A modification according to Fig. 7 differs in the design of the protrusion. The protrusion 10' comprises at least one, preferably more than one circumferentially extending rows of pimples or small bulges. A row of pimples 10a, 10b, 10c extends along the full width of a wall portion 9a in the circumferential direction. The number and size of the pimples and the number of rows may be selected according to desired holding force. It shall be noted that the lowermost row may be located close to the bottom 7a of the first recess 7. The uppermost row may have a distance from the upper edge of the wall portion 9 to facilitate insertion of the engagement portion 30.

A further modification is shown in Fig. 8 in which the protrusion 10' comprises a structured area of small projections 10d. The projections 10d can be a protruding area of local roughness or can be a field of pimples or rounded pyramids that are arranged close to each other, for example that may be regularly arranged in an array-like manner.

A further modification of the screw element is shown in Fig. 9. It differs from the previous modifications by the design of the drive portion. The drive portion 6' has a spiral shape like a drive known under the name Mortorq^{®}. The screw element 1 according to this modified embodiment includes a spherical head 4' that also has a spherical outer surface at a free upper end 5'. In the embodiment, the head is substantially entirely spherical. A recess 7' for the screwdriver is formed by a groove 71 provided in the spherical surface of the head 4' in such a manner that an inner wall 9' of the groove 71 gives the upper head portion a shape of arms 72 that are bent in a spiral-like manner around a center of the upper free end 5'. In the embodiment shown, the recess 7' includes four arms 72. The sections of the groove 71 forming the arms 72 also provide the drive grooves 8'. Each arm 72 comprises a first wall portion 9a' and an opposite second wall portion 9b' wherein the first wall portion 9a' is slightly concavely formed or substantially flat and the second wall portion 9b' is convexly formed, thus providing the spiral-like shape of the arm 72. On the first wall portion the protrusion 10', preferably substantially in the center of the wall portion 9a', is provided. The protrusion 10' may comprise, for example, several pimples that are arranged in a vertical line in the axial direction of the screw element. However, other arrangements of the protrusion 10' may also be selected.

The corresponding engagement portion (not shown) of the screwdriver has a shape that fits into the groove 71. By means of this shape, an engagement surface between the engagement portion of the screwdriver and the drive portion of the screw element can be enhanced and thus the engagement may be more robust compared to usual polygon engagement shapes. Therefore, the loads that can be transferred onto the head 4' may be higher.

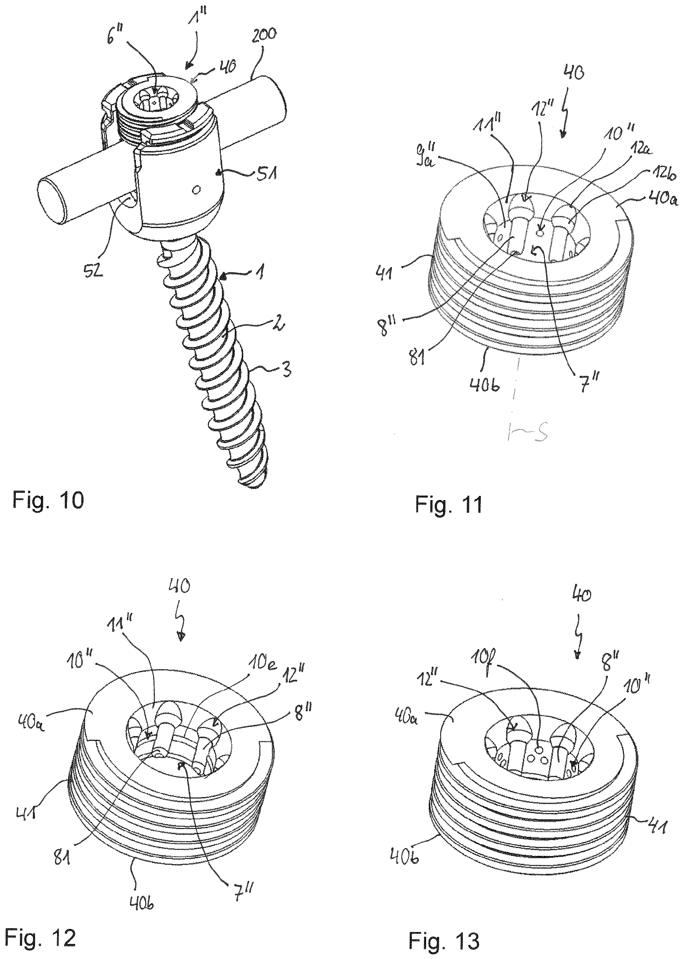

A second embodiment of a screw element is explained with reference to Figs. 10 and 11. Parts and portions that are the same or substantially the same as the previous embodiments are marked with the same numerals and the descriptions thereof will not be repeated. In the embodiment of Figs. 10 and 11 the screw element 1" is a set screw 40 that is used in a polyaxial bone anchoring device 50. The polyaxial bone anchoring device 50 is shown only in an exemplary manner, many different designs of such polyaxial bone anchoring devices may be contemplated.

In greater detail, the polyaxial bone anchoring device 50 includes a bone anchor in the form of the screw element 1 with the shank 2, a spherical segment-shaped head (not shown) and a drive portion 6, 6' in the head according to the previously described first embodiment and its modifications. However, also a bone anchor with a conventional drive portion such as a torx-shaped drive portion or a polygon-shaped drive portion in the head may be used. The screw element 1 is pivotably connected to a receiving part 51 that includes a seat to hold the head of the screw element 1 in a ball and socket manner. A pressure element (not shown) may also be provided to exert pressure onto the head. The receiving part 51 also includes a substantially U-shaped recess 52 that is configured to receive a rod 200 therein. The rod 200 may be connected to a plurality of bone anchoring devices. To lock the rod 200 in a receiving part 51 and a pivot position of the head relative to the receiving part 51, the screw element 1" according to the second embodiment which is in the form of the set screw 40 is used.

The set screw 40 comprises a first end or upper end 40a and a second end or lower end 40b. An external thread 41 of the set screw 40 cooperates with a corresponding internal thread in the receiving part 51. At a distance from the first end 40a a first recess 7" is formed. The first recess 7" comprises substantially cylindrical inner wall portions 9a" that are separated from each other by longitudinal drive grooves 8" the bottom 81 of which provides an abutment for the drive portion 30 of the screwdriver 20 in the axial direction. The bottom of the recess 7" may be open towards the lower end 40b. The second recess 11" may be slanted like in the first embodiment. At positions corresponding to the positions of the drive grooves 8" guide grooves 12" are formed in the slanted second recess 11". The guide grooves 12" have a greater width than the corresponding drive grooves 8", respectively. In greater detail, the guide grooves 12" may have a first groove portion 12a adjacent to the upper end 40a that is rounded and has a gradually increasing width in the direction of the first recess 7". Furthermore, the guide grooves 12" may comprise an intermediate section of inclined shoulder 12b that narrows towards the drive grooves 8". The intermediate section 12b and the first portion 12a of the guide grooves may form pocket-like recesses that catch and guide the engagement portion 30 of the screwdriver 20 into the guide grooves 8".

On the wall portions 9a" a protrusion 10" is provided. In the embodiment, the protrusion 10" may consist of a single bulge or a single pimple provided substantially in the center of each wall portion 9a" in the circumferential direction. The protrusion 10" may be closer to the second recess 11" than to the bottom 81 of the drive grooves 8" in the axial direction. However, the protrusion 10" can also be, for example, in the middle of the wall portion 9" or at any other position.

In use, the screw element 1" in the form of the set crew 40 is engaged by the engagement portion 30 of the screwdriver 20. By means of the protrusion 10" the engagement portion is firmly clamped in the first recess 7". This facilitates the insertion of the screw element 1" into the U-shaped recess 52 of the receiving part 51 of the polyaxial bone anchoring device and may facilitate improve the transfer of the force to tighten the set screw 40. Once the head of the bone anchor and the rod are locked, further adjustments may become necessary. To make such adjustments, the set screw 40 must be loosened and tightened again after correcting the angular position of the receiving part 51 relative to the head or after correcting the position of the rod 200.

Fig. 12 shows a modification of the screw element 40 wherein the protrusion 10‴ is formed as a circumferential rib 10e that extends entirely through the wall portion 9" in the circumferential direction.

In a further modification shown in Fig. 13, the protrusion 10‴ comprises bulges or pimples 10f that are arranged in a triangular manner substantially in the center of the wall portion 9a". It shall be noted that other structures of the protrusion 10" may also be implemented, for example such as shown in Figs 6 to 9.

Fig. 14 shows a first clinical application in which the screw element 1 according to the first embodiment is screwed with the screwdriver 20, the engagement portion 30 of which engages the drive portion 6, into the pedicle of a vertebra 500. In Fig. 15, a polyaxial bone anchoring device is shown may that comprise the screw element 1' with the drive portion 6, 6' according to the first embodiment. For fixing the rod 200, set screw 40 according to the second embodiment may be used. The polyaxial bone anchoring device may be a bottom-loading polyaxial bone anchoring device, i.e., where the head 4, 4' of the screw element 1 can be inserted into the receiving part from the bottom of the receiving part 51. Thus, the screw element 1 can be inserted first into the bone as shown in Fig. 14 and the receiving part 51 can be mounted thereafter. The drive portion 6, 6' according to the screw element 1 of the first embodiment is particularly useful for such in-situ placement of the screw element 1 into the vertebra prior to mounting the receiving part 51. However, the screw element 1 according to the first embodiment can also be preassembled with the receiving part prior to insertion into bone.

A further clinical application is shown in Fig. 16. Fig. 16 depicts a bone plate 60 that may be used with the screw element 1 of the first embodiment according to Figs. 1 to 9, for example in orthopaedic and trauma surgery to immobilize broke bone parts. The spherical head 4, 4' of the screw element 1 can assume within a hole 61 of the bone plate 60 various angles. Alternatively, the head may have a shape that limits positioning of the screw element, 1 for example, to a fixed angle with respect to the bone plate 60. When implanting the bone plate 60, the screwdriver 20 engages with the engagement portion 30 and the drive portion 6, 6' to fix the bone plate 60 with the screw element 1 to the bone. In a still further modification, a locking element (not shown) in the form of a set screw may be provided in the holes of the bone plate 60 to prevent pull-out of the screw elements 1. Such a locking element may have a drive portion 6" as shown in Figs. 10 to 13. Such a locking element may have at its lower side a recess for receiving a portion of the head 4, 4' of the screw element 1.

Modifications of the screw element are conceivable. Without restriction, such modifications are for example the following. The second recess 11 can be omitted. The bottom of the first recess 7, 7" can be open or closed. For example, if the screw element 1 is a cannulated bone anchor, the bottom of the first recess may be open. In the embodiments shown, an even number of drive grooves 8, 8" are shown and each drive groove 8, 8" is positioned opposite to another drive groove in the drive portions 6, 6', 6". However, an odd number of drive grooves may also be contemplated. The design of the protrusion may vary between the wall portions within one drive portion.

In addition, instead of the star- or torx-shape of the first recess, a polygonal shape of the first recess may also be contemplated. In such a case, the corners of the polygon may be considered the drive grooves.

The screw element according to the first embodiment may also include a bone nail. The head may have other external shapes than spherical, for example cylindrical or otherwise shaped. The polyaxial bone anchoring device can also be a mono-planar bone anchoring device where the screw element can pivot only in a single plane.

The screw element of the second embodiment may also have the design of the drive portion as shown in Figs. 6 to 9.

## Claims

1. A screw element for use in spinal, orthopaedic or trauma surgery,
the screw element (1, 1") including a screw axis (S) and a screw thread (3, 41) and
a drive portion (6, 6', 6") comprising a recess (7, 7', 7") with a wall (9, 9', 9") that defines a main contour configured to engage with a screwdriver, the wall further defining a plurality of drive grooves (8, 71, 8"),
wherein the drive grooves (8, 71, 8") are spaced apart from each other in a circumferential direction around the screw axis (S); and
wherein a wall portion (9a, 9a', 9a") of the recess (7, 7', 7") comprises a protrusion (10, 10', 10") that protrudes from the main contour into the recess (7, 7', 7").

2. The screw element of claim 1, wherein the protrusion (10, 10', 10") has a size such that it permits to insert an engagement portion (30) of a screwdriver (20) while it reduces a radial play (13) of the engagement portion (30) in the recess (7, 7', 7").

3. The screw element of claim 1 or 2, wherein the protrusion (10, 10', 10") has a size such that it is configured to clamp the engagement portion (30) by friction.

4. The screw element of one claims 1 to 3, wherein a portion of the screw element (1, 1") comprising the drive portion (6, 6', 6") is sized and/or shaped such that it is only manufacturable by an additive manufacturing method.

5. The screw element of one of claims 1 to 4, wherein the protrusion (10, 10', 10") comprises a geometrical shape and/or a local roughness.

6. The screw element of one of claims 1 to 5, wherein the protrusion (10, 10', 10") is locally distinct on the wall portion (9a, 9a', 9a").

7. The screw element of one of claims 1 to 6, wherein the protrusion (10, 10', 10") comprises one or more bulges, pimples, ribs, preferably extending in a circumferential direction, a structured roughness or mixtures thereof.

8. The screw element of one of claims 1 to 7, wherein the drive portion (6, 6") comprises in a top view a star-like shape wherein the drive grooves (8) comprise axially extending grooves with cylindrical wall portions (9a, 9a") in-between or wherein the drive portion comprises a polygonal shape and wherein the drive grooves are the corners of the polygon.

9. The screw element of claim 8, wherein each of the wall portions (9a, 9a") between the drive grooves (8, 8") comprise the protrusion (10, 10").

10. The screw element of one of claims 8 or 9, wherein the recess (7, 7") is a first recess and wherein the screw element comprises a second recess (11, 11") between the free end (5, 40a) which tapers from the free end towards the first recess and which comprises guide grooves (12, 12") at positions corresponding to the positions of the drive grooves (8, 8").

11. The screw element of one of claims 1 to 7, wherein the drive portion comprises a recess (7') formed by a groove (71) that provides a spiral-like shape and wherein an inner wall (9') of the recess comprises a first wall portion (9a') that is concavely-shaped or flat and that has the protrusion (10') whereas a second wall portion (9b') opposite to the first wall portion (9a') does not have the protrusion (10').

12. The screw element of one of claims 1 to 11, wherein the screw element (1) is a bone screw comprising a shank (2) with a bone thread (3) in at least a portion thereof and with a head (4, 4') having a free end (5, 5') opposite to the shank and wherein the drive portion (6, 6') is provided in the head, preferably at the free end of the head.

13. The screw element of one of claims 1 to 12, wherein the screw element (1") comprises a set screw (40) configured to be used as a locking element for a polyaxial bone screw (50) or a locking element for a bone plate (60).

14. A system of a screw element of one of claims 1 to 13 and a screwdriver, wherein the screwdriver (20) comprises an engagement portion (30) for engaging the recess (7, 7'), preferably wherein the engagement portion (30) has outer wall portions that have a shape corresponding to the shape of the wall portions of the recess without the protrusion.

15. A method of manufacturing a screw element of one of claims 1 to 13, the method comprising manufacturing by an additive manufacturing method at least a portion of the screw element (1, 1") comprising the drive portion (6, 6', 6") with the steps
providing computer data of a three-dimensional (3D) model of the portion of the screw element,
manufacturing the portion of the screw element layer by layer from a building material according to a cross-section of the portion of the screw element in the corresponding layer;
preferably wherein the additive manufacturing method comprises one of laser sintering laser melting or electron beam melting.

## Amended claims

### Amended claims in accordance with Rule 137(2) EPC.

1. A screw element for use in spinal, orthopaedic or trauma surgery,
the screw element (1, 1") including a screw axis (S) and a screw thread (3, 41) and
a drive portion (6, 6', 6") comprising a recess (7, 7', 7") with a wall (9, 9', 9") that defines a main contour configured to engage with a screwdriver, the wall further defining a plurality of drive grooves (8, 71, 8"),
wherein the drive grooves (8, 71, 8") are spaced apart from each other in a circumferential direction around the screw axis (S);
wherein a wall portion (9a, 9a', 9a") of the recess (7, 7', 7") comprises a protrusion (10, 10', 10") that protrudes from the main contour into the recess (7, 7', 7"), and
wherein a portion of the screw element (1, 1") comprising the drive portion (6, 6', 6") is sized and/or shaped such that it is only manufacturable by an additive manufacturing method.

2. The screw element of claim 1, wherein the protrusion (10, 10', 10") has a size such that it permits to insert an engagement portion (30) of a screwdriver (20) while it reduces a radial play (13) of the engagement portion (30) in the recess (7, 7', 7").

3. The screw element of claim 1 or 2, wherein the protrusion (10, 10', 10") has a size such that it is configured to clamp the engagement portion (30) by friction.

4. The screw element of one of claims 1 to 3, wherein the protrusion (10, 10', 10") comprises a geometrical shape and/or a local roughness.

5. The screw element of one of claims 1 to 4, wherein the protrusion (10, 10', 10") is locally distinct on the wall portion (9a, 9a', 9a").

6. The screw element of one of claims 1 to 5, wherein the protrusion (10, 10', 10") comprises one or more bulges, pimples, ribs, preferably extending in a circumferential direction, a structured roughness or mixtures thereof.

7. The screw element of one of claims 1 to 6, wherein the drive portion (6, 6") comprises in a top view a star-like shape wherein the drive grooves (8) comprise axially extending grooves with cylindrical wall portions (9a, 9a") in-between or wherein the drive portion comprises a polygonal shape and wherein the drive grooves are the corners of the polygon.

8. The screw element of claim 7, wherein each of the wall portions (9a, 9a") between the drive grooves (8, 8") comprise the protrusion (10, 10").

9. The screw element of one of claims 7 or 8, wherein the recess (7, 7") is a first recess and wherein the screw element comprises a second recess (11, 11") between the free end (5, 40a) which tapers from the free end towards the first recess and which comprises guide grooves (12, 12") at positions corresponding to the positions of the drive grooves (8, 8").

10. The screw element of one of claims 1 to 6, wherein the drive portion comprises a recess (7') formed by a groove (71) that provides a spiral-like shape and wherein an inner wall (9') of the recess comprises a first wall portion (9a') that is concavely-shaped or flat and that has the protrusion (10') whereas a second wall portion (9b') opposite to the first wall portion (9a') does not have the protrusion (10').

11. The screw element of one of claims 1 to 10, wherein the screw element (1) is a bone screw comprising a shank (2) with a bone thread (3) in at least a portion thereof and with a head (4, 4') having a free end (5, 5') opposite to the shank and wherein the drive portion (6, 6') is provided in the head, preferably at the free end of the head.

12. The screw element of one of claims 1 to 11, wherein the screw element (1") comprises a set screw (40) configured to be used as a locking element for a polyaxial bone screw (50) or a locking element for a bone plate (60).

13. A system of a screw element of one of claims 1 to 12 and a screwdriver, wherein the screwdriver (20) comprises an engagement portion (30) for engaging the recess (7, 7'), preferably wherein the engagement portion (30) has outer wall portions that have a shape corresponding to the shape of the wall portions of the recess without the protrusion.

14. A method of manufacturing a screw element of one of claims 1 to 12, the method comprising manufacturing by an additive manufacturing method at least a portion of the screw element (1, 1") comprising the drive portion (6, 6', 6") with the steps
providing computer data of a three-dimensional (3D) model of the portion of the screw element,
manufacturing the portion of the screw element layer by layer from a building material according to a cross-section of the portion of the screw element in the corresponding layer;
preferably wherein the additive manufacturing method comprises one of laser sintering laser melting or electron beam melting.
